# EUROPEAN PATENT APPLICATION

(11) **EP 3 222 325 A1**
(43) Date of publication of application: **27.09.2017**
(21) Application number: 16162472.1
(22) Date of filing: 25.03.2016
(51) Int. Cl.: A61Q 1/06, A61K 8/86

(54) **LIPSTICK FORMULATIONS**

(71) Applicant: Solvay Specialty Polymers Italy S.p.A., 20021 Bollate (MI) (IT)
(72) Inventor: BERTASA, Anna Maria, 20020 CESATE (MI) (IT); PETRICCI, Silvia Rita, 20091 BRESSO (MI) (IT)
(74) Representative: Benvenuti, Federica

(57) **Abstract**

The present invention relates to the use of functional (per)fluoropolyether polymers as additive in lipstick formulations.

## Description

### Technical Field

The present invention relates to the use of functional (per)fluoropolyether polymers as additive in lipstick formulations.

### Background Art

Lipsticks have been used for many years to put colour and gloss to the lips to draw charm, as they are recognized as one of the makeup cosmetics with the highest cosmetic effect.

Lipsticks are typically prepared in form of water-in-oil emulsions or anhydrous formulations.

While water-in-oil emulsions provide a better moisturizing effect to the lips, they raise more concerns as to their durability and storage conditions because water can lead to the development of a high microbial charge.

On the other hand, anhydrous formulations tend to be not hydrating and moisturizing for the lips, such that cracks tend to appear on the lips when the lipstick is worn for several hours.

When the anhydrous formulation is applied to the lip surface, friction melts it briefly and allows for transfer the pigment. Then, the material cools and re-forms creating a film that sticks to the surface of the lips due to hydrophobic interactions.

For the manufacture of anhydrous formulations, typically fats and/or oils, together with pigments and/or lakes, and other non-aqueous ingredients, are added to a wax base, which is first melted to enable the ingredients to be thoroughly mixed and is then cast into moulds, which after cooling, provide the shaped moulded product.

(Per)fluoropolyether polymers have been disclosed in the art as additives in personal care compositions.

For example, EP 0196904 A (AUSIMONT S.P.A.) discloses compositions for cosmetic and dermatology comprising a liquid perfluoropolyether dispersed in an oil/water or water/oil emulsion, or in a solid phase able to form a gel suspended in a liquid organic phase. Non-functionalized (per)fluoropolyethers such as notably Fomblin^{®} Y are disclosed in the examples. Examples of the applications include - among the others - decorative cosmetics, notably lipsticks and lip-glosses, in which an improvement of both flowability and glossiness is said to be obtained.

EP 0390206 A (AUSIMONT S.P.A.) discloses a stable emulsion comprising: a) perfluoropolyethers having perfluoroalkyl end groups; b) conventional surfactants and c) a continuous phase consisting of glycerol or of a polyhydroxylated compound containing at least three hydroxyl groups dissolved in a hydrophilic solvent and/or water. Non-functionalized (per)fluoropolyethers such as notably Fomblin^{®} HC are disclosed in the examples. Examples of the applications include decorative cosmetic, notably lipsticks and lip-glosses, in which an improvement of both flowability and glossiness is said to be obtained.

EP 0494412 A (AUSIMONT S.P.A.) discloses a stable emulsion of perfluoropolyethers having perfluoroalkyl end groups and fat substances, said emulsion being for use in cosmetology and dermatology. Non-functionalized (per)fluoropolyethers such as notably Fomblin^{®} HC are disclosed in the examples. Among the cosmetic applications, anhydrous cosmetics such as lipsticks and sticks are cited. The anhydrous nature of the emulsion, or in any case, the presence therein of little water amounts, makes biologically more stable the cosmetic and dermatological products prepared with the emulsion, and secures a higher persistence of said products on the skin.

EP 1074243 A (AUSIMONT S.P.A.) discloses a base composition (premix) comprising A) a perfluoropolyether phosphate (notably FOMBLIN(R) HC/P2-1000), B) a solvent and C) water. Concentrated compositions are said to be suitable in the preparation of cosmetic compositions, including compositions for make-up.

None of the abovementioned patent applications discloses or suggests the use of functionalized (per)fluoropolyethers as additives in lipstick formulations.

### Summary of invention

The Applicant faced the problem to provide a lipstick composition that is aesthetically appealing and easy to spread, while showing good covering power, durability and adhesion to the lips.

The Applicant has surprisingly found that when a functional (per)fluoropolyether polymer is used as additive, lipstick formulations having the abovementioned advantages can be provided.

In a first aspect, the present invention relates to a method for colouring the lips, which comprises applying to the lips a lipstick formulation [formulation F], said formulation F comprising at least one functional (per)fluoropolyether polymer [polymer P] and a pigmented base.

In a second aspect, the present invention relates to the use of a functional (per)fluoropolyether polymer [polymer P] as additive in a lipstick formulation [formulation F].

More in particular, said polymer P comprises a (per)fluoropolyether chain [chain (R_{pf})] having two chain ends [chains (Rₑ)], wherein at least one chain (Rₑ) bears at least one functional group.

The Applicant has surprisingly found that the properties covering power, durability and adhesion are improved when said polymer P is used in an amount lower than 1wt.% based on the total weight of the formulation F.

Without being bound by any theory, the Applicant believes that when used in an amount of 1wt.% or greater, the functional PFPE interacts with the other ingredients inducing a slight separation or segregation of the same, which results in an impairment of the properties of the formulation F.

In a third aspect, the present invention relates to a lipstick formulation [formulation F] comprising
from 0.001 to less than 1 wt.% based on the total weight of formulation F of at least one polymer P as defined above and
up to 100 wt.% based on the total weight of formulation F of a pigmented base.

In a fourth aspect, the present invention relates to a kit comprising a container having a lid containing formulation F as defined above.

### Description of embodiments

For the purposes of the present description:
- the use of parentheses around symbols or numbers identifying the formulae, for example in expressions like "polymer (P)", etc., has the mere purpose of better distinguishing the symbol or number from the rest of the text and, hence, said parenthesis can also be omitted;
- the acronym "PFPE" stands for "(per)fluoropolyether" and, when used as substantive, is intended to mean either the singular or the plural from, depending on the context;
- the prefix "(per)" in the term "(per)fluoropolyether" is intended to indicate that the polyether can be fully or partially fluorinated.

Preferably, said chain (R_{pf}) is a chain of formula:

-O-D-(CFX^{#})_{z1}-O(R_{f})(CFX^{*})_{z2}-D^{*}-O-

wherein
z1 and z2, equal or different from each other, are equal to or higher than 1;
X^{#} and X^{*}, equal or different from each other, are -F or -CF₃, provided that when z1 and/or z2 are higher than 1, X^{#} and X^{*} are -F; D and D*, equal or different from each other, are an alkylene chain comprising from 1 to 6 and even more preferably from 1 to 3 carbon atoms, said alkyl chain being optionally substituted with at least one perfluoroalkyl group comprising from 1 to 3 carbon atoms;
(R_{f}) comprises, preferably consists of, repeating units R°, said repeating units being independently selected from the group consisting of:
   (i) -CFXO-, wherein X is F or CF₃;
   (ii) -CFXCFXO-, wherein X, equal or different at each occurrence, is F or CF₃, with the proviso that at least one of X is -F;
   (iii) -CF₂CF₂CW₂O-, wherein each of W, equal or different from each other, are F, Cl, H;
   (iv) -CF₂CF₂CF₂CF₂O-;
   (v) -(CF₂)ⱼ-CFZ-O- wherein j is an integer from 0 to 3 and Z is a group of general formula -O-R(_{f-a})-T, wherein R(_{f-a}) is a fluoropolyoxyalkene chain comprising a number of repeating units from O to 10, said recurring units being chosen among the following : -CFXO- , -CF₂CFXO-, -CF₂CF₂CF₂ O-, -CF₂CF₂CF₂CF₂O-, with each of X being independently F or CF₃ and T being a C₁-C₃ perfluoroalkyl group.

Preferably, z1 and z2, equal or different from each other, are from 1 to 10, more preferably from 1 to 6 and even more preferably from 1 to 3.

More preferably, D and D*, equal or different from each other, are a chain of formula -CH₂-, -CH₂CH₂- or -CH(CF₃)-.

Preferably, chain (R_{f}) complies with the following formula:

(R_{f}-I) -[(CFX¹O)_{g1}(CFX²CFX³O)_{g2}(CF₂CF₂CF₂O)_{g3}(CF₂CF₂CF₂CF₂O)_{g4}]-

wherein
- X¹ is independently selected from -F and -CF₃,
- X², X³, equal or different from each other and at each occurrence, are independently -F, -CF₃, with the proviso that at least one of X is -F;
- g1, g2 , g3, and g4, equal or different from each other, are independently integers ≥0, such that g1+g2+g3+g4 is in the range from 2 to 300, preferably from 2 to 100; should at least two of g1, g2, g3 and g4 be different from zero, the different recurring units are generally statistically distributed along the chain.

More preferably, chain (R_{f}) is selected from chains of formula:

(R_{f}-IIA)-[(CF₂CF₂O)ₐ₁(CF₂O)ₐ₂]-

wherein:
- a1 and a2 are independently integers ≥ 0 such that the number average molecular weight is between 400 and 10,000, preferably between 400 and 5,000; both a1 and a2 are preferably different from zero, with the ratio a1/a2 being preferably comprised between 0.1 and 10;

(R_{f}-IIB)-[(CF₂CF₂O)_{b1}(CF₂O)_{b2}(CF(CF₃)O)_{b3}(CF₂CF(CF₃)O)_{b4}]-

wherein:
b1, b2, b3, b4, are independently integers ≥ 0 such that the number average molecular weight is between 400 and 10,000, preferably between 400 and 5,000; preferably b1 is 0, b2, b3, b4 are > 0, with the ratio b4/(b2+b3) being ≥1;

(R_{f}-IIC)-[(CF₂CF₂O)_{c1}(CF₂O)_{c2}(CF₂(CF₂)_{cw}CF₂O)_{c3}]-

wherein:
cw = 1 or 2;
c1, c2, and c3 are independently integers ≥ 0 chosen so that the number average molecular weight is between 400 and 10,000, preferably between 400 and 5,000; preferably c1, c2 and c3 are all > 0, with the ratio c3/(c1+c2) being generally lower than 0.2;

(R_{f}-IID)-[(CF₂CF(CF₃)O)_{d}]-

wherein:
d is an integer > 0 such that the number average molecular weight is between 400 and 10,000, preferably between 400 and 5,000;

(R_{f}-IIE)-[(CF₂CF₂C(Hal*)₂O)ₑ₁-(CF₂CF₂CH₂O)ₑ₂-(CF₂CF₂CH(Hal*)O)ₑ₃]-

wherein:
- Hal*, equal or different at each occurrence, is a halogen selected from fluorine and chlorine atoms, preferably a fluorine atom;
- e1, e2, and e3, equal to or different from each other, are independently integers ≥ 0 such that the (e1+e2+e3) sum is comprised between 2 and 300.

Still more preferably, chain (R_{f}) complies with formula (R_{f}-III) here below:

(R_{f}-III)-[(CF₂CF₂O)ₐ₁(CF₂O)ₐ₂]-

wherein:
- a1, and a2 are integers > 0 such that the number average molecular weight is between 400 and 10,000, preferably between 400 and 5,000, with the ratio a1/a2 being generally comprised between 0.1 and 10, more preferably between 0.2 and 5.

Preferably, when only one chain (Rₑ) bears at least one functional group, the other chain (Rₑ) bears a neutral group selected from -CF₃, -C₂F₅, -C₃F ₇, -CF₂Cl, -CF₂CF₂Cl and -C₃F₆Cl. In this case, polymer P is also referred to as "mono-functional polymer P".

Preferably, both chains (Rₑ) bear at least one functional group. In this case, polymer P is also referred to as "bi-functional polymer P". Preferably, the functionality of the bi-functional polymer P, i.e. the number of functional groups, is at least equal to 1.80, more preferably at least equal to 1.85 and still more preferably at least equal to 1.94. The functionality (F) can be calculated for example as disclosed in EP 1810987 A (SOLVAY SOLEXIS S.P.A.) .

Embodiments wherein both chains (Rₑ) bear at least one functional group are preferred. More preferably, both chains (Rₑ) bear the same functional group.

Preferred functional groups are selected from the group consisting of hydroxy group and phosphate group.

According to a preferred embodiment, both chains (Rₑ) bear one hydroxy group or both chains (Rₑ) bear one phosphate group.

Preferably, formulation F comprises polymer P in an amount of from 0.001 to less than 1 both chains (Rₑ) bear one hydroxy group or both chains (Rₑ) bear one phosphate group. %, more preferably from 0.05 to 0.5 wt.% based on the total weight of the formulation F.

Preferably, formulation F is a dermatologically acceptable formulation.

Preferably, formulation F has a dropping point in the range from about 50 to 75°C, more preferably from 60 to 70°C. Dropping point can be measured using standard methods originally developed for waxes, for example according to ASTM D566 or D2265, or using a dropping point thermomether with Ubbelhode pattern.

Preferably, formulation F is an anhydrous formulation.

However, according to some embodiments, formulation F can include water. When present, the amount of water in formulation F can be determined by the skilled person depending on the nature and amount of the other ingredients.

Preferably, formulation F is solid at room temperature, i.e. at about 25°C.

Preferably, the pigmented base comprises ingredients selected from the group comprising: dyes and colorants, moisturizers, UV absorbers, fillers, antioxidants, conditioning agents, thickeners.

Typically, dyes and colorants are selected in the group comprising metal oxides (e.g. iron oxides, chromium oxides, titanium dioxides, aluminium hydroxides and the like), mica, and mixtures thereof.

Preferably, dyes and colorants are present in formulation F in an amount of from 1 to 30 wt.%, more preferably from 5 to 25 wt.% based on the total weight of the formulation F.

Typically, moisturizing agents are selected in the group comprising amino acids, lanolin, botanical extracts (e.g., from *Athea officinalis, Arnica montana, Betula alba, Botrago officinalis, Ruscus aculeatus, Calendula officinalis,* and the like), waxes and butter (e.g. from Euphorbia cerifera, carnuba wax, cocoa butter, shea butter, and the like), oils (e.g., cardamom oil, castor oil, chamomile oil, clary oil, cononut oil, olive oil, rice bran oil and the like), esters of fatty acids (e.g. esters of stearic acid, oleic acid, lanolin acid, myristic acid, palmitic acid and the like) including esters of fatty acids with polyethylene glycol (PEG) or propylene glycol, and mixture thereof.

Preferably, moisturizing agents are present in formulation F in an amount of from 5 to 50 wt.%, more preferably from 10 to 40 wt.% based on the total weight of the formulation F.

Typically, UV absorbers are selected from chemical and physical sun-blocks. Non limiting examples of chemical sun-blocks include para-aminobenzoic acid (PABA) and derivatives thereof; benzophenones; cinnamates (e.g. octyl methoxycynnamate, isoamyl p-methoxycynnamate, pentaerythrytyl tetra-di-tertbutyl hydroxycynnamate); salicylates. Non limiting examples of physical sub-blocks include kaolin, talc and metal oxides (e.g., titanium dioxide and zinc oxide), and mixture thereof.

Preferably, UV absorbers are present in formulation F in an amount of from 0.01 to 1 wt.%, more preferably from 0.02 to 0.5 wt.% based on the total weight of the formulation F.

Typically, fillers are selected from the groups comprising inorganic salts (e.g., boron nitride).

Preferably, fillers are present in formulation F in an amount of from 0.1 to 10 wt.%, more preferably from 1 to 5 wt.% based on the total weight of the formulation F.

Typically, antioxidants are selected from the groups comprising tocopherol and derivatives thereof (e.g., tocophereth-5, tocophereth-10, tocopheryl acetate, tocopheryl linoleate, tocopheryl nicotinate, tocopheryl succinate and the like), BHA, BHT, hydroquinone, organic and inorganic sodium salts (e.g. sodium ascorbate, sodium bisulfite, and the like), and mixture thereof.

Preferably, antioxidants are present in formulation F in an amount of from 0.1 to 10 wt.%, more preferably from 0.5 to 3 wt.% based on the total weight of the formulation F.

Typically, conditioning agents are selected from the group comprising tocopheryl acetate, shea butter, polymers of polyethylene and methicone, tocopherol, glycerine, hyaluronic acid, cocamidopropyl betaine, pentaerythrityl tetraisostearate, bis-diglyceryl polyacyladipate-2, and mixtures thereof.

Preferably, conditioning agents are present in formulation F in an amount of from 2 to 40 wt.%, more preferably from 5 to 30 wt.% based on the total weight of the formulation F.

Typically, thickeners are selected from substances that can increase or control the viscosity of a composition. Preferably, thickeners are selected from the group comprising hydrogenated polyisobutene, trihydroxystearin and mixtures thereof, carboxylic acid polymers, crosslinked acrylate polymers, polyacrylamide polymers, polysaccharides, gums, inorganic agents (e.g. calcium aluminium borosilicate, calcium sodium borosilicate, ozokerite, and the like), and mixtures thereof.

Preferably, thickeners are present in formulation F in an amount of from 2 to 40 wt.%, more preferably from 5 to 30 wt.% based on the total weight of the formulation F.

A variety of additional ingredients can be further added to the pigmented base of formulation F in order to modify its aesthetic properties, melting characteristics, viscosity of the adhesion profile, stability and also to make its production easier.

Additional ingredients that can be added to the pigmented base of formulation F can be selected for example from the group comprising:
flavouring agents; botanical extracts; anti-microbial agents; anti-irritants agents; chelating agents; preservatives; pH adjusters; humectants;
fragrances; essential oils; lubricants; solidifiers (e.g. waxes such as beeswax, carnauba wax or candelilla wax); viscosity modifiers (e.g. polyethylene); and silicone containing compounds.

Each of these additional ingredients can be present in formulation F in suitable amounts, notably from 0.001 to 5 wt.% based on the total weight of the formulation F.

Formulation F can be prepared according to known techniques. For example, a step of uniformly blending or kneading together the above described ingredients into a homogeneous mixture is performed.

Preferably, a heating step of the ingredients is performed before blending or kneading. The heating temperature can be selected by the skilled person depending on the starting ingredients used to prepare the formulation F. Different ingredients are preferably heated at different temperatures before blending or kneading. Suitable temperatures are typically below 100°C, preferably from 50 to 98°C.

Preferably, the homogeneous mixture thus obtained is poured into a suitable mould having the desired final shape.

A cooling step is then performed in order to obtain formulation F in the form of a solid formulation.

Formulation F is preferably formed into an elongated shape or stick.

Preferably, formulation F is provided inside a container having a lid.

The step of applying formulation F to the lips can be performed by applying the solid formulation F on the lips directly or by means of an applicator, for example a little brush.

The invention will be hereinafter illustrated in greater detail by means of the Examples contained in the following Experimental Section; the Examples are merely illustrative and are by no means to be interpreted as limiting the scope of the invention.

### Experimental Section

### Materials

The following were obtained from Solvay Specialty Polymers Italy S.p.A.:
Fomblin^{®} HC/R PFPE: non-functional perfluoropolyether polymer having perfluoroalkyl groups at its chain ends
Fomblin^{®} HC/OH-1000 PFPE: functional perfluoropolyether polymer having -OH groups at both its chain ends
Fomblin^{®} HC/P2-1000 PFPE: functional perfluoropolyether polymer having phosphate groups at both its chain ends

The following were obtained from LCM TRADING S.p.A. RADIA 7750
UNIPURE YELLOW LC^{®} 182 (dispersion 40% in RADIA 7750) UNIPURE RED LC^{®} 3075 (dispersion 30% in RADIA 7750) PERMULGIN 3283

Also the other ingredients were commercially available:
FANCOL POLYISO® 800-CG from Pharmacosm Polli
A402 TUDOR ROSEWOOD (dispersion 40% in RADIA 7750) from
KINGFISHER / DGS
CRODAMOL™ PTIS and ARLACEL™ 582 from CRODA
TINOGARD® TT and VITAMIN E ACETATE from BASF/EUROTRADING
PERFORMALENE™ 500 from Castelli
FANCOL® VB from Pharmacosm Polli
PLANTASENSE® CRYSTOLIVE WAX from Clariant
CARNAUBA WAX from Caldic Italia
SOFTISAN® 649 from EIGENMANN & VERONELLI
TOCOTRIENOL 70% from GIELLEPI CHEMICALS
RONAFLAIR® BORONEIGE® SF15 from MERCK/COMETECH

### Preparation of base formulation 1C (comparative)

A dry lipstick base formulation (formulation 1C) was prepared by mixing the ingredients in the amounts as listed in the following Table:

**Table**

| **Phase** | **Ingredient (commercial name)** | **INCI Name** | **% w**/**w** |
|---|---|---|---|
| A | RADIA 7750 | Isoamyl Laurate | 0.33 |
| | FANCOL POLYISO® 800-CG | Hydrogenated Polyisobutene | 15.00 |
| | A402 TUDOR ROSEWOOD | Irox oxides (Cl 77491 and Cl 77718) | 8.10 |
| | UNIPURE YELLOW LC® 182 | Iron oxides (Cl 77492) | 5.00 |
| | UNIPURE RED LC® 3075 | Cl 15850 and Aluminium Hydroxide | 6.30 |
| B | CRODAMOL™ PTIS | Pentaerythrytyl Tetraisostearate | 15.00 |
| | TINOGARD® TT | Pentaerythrytyl Tetra-di-t-butyl Hydroxhycinnamate | 0.05 |
| | PERFORMALENE™ 500 | Polyethylene | 2.00 |
| C | FANCOL® VB | Mixture of Limnanthes Alba (Meadowfoam) Seed Oil and Butyrospermum Parkii (Shea) Butter Extract | 15.00 |
| | PLANTASENSE® CRYSTOLIVE WAX | Olea Europaea Oil Unsaponafiables | 5.00 |
| | CARNAUBA WAX | Copernicia Prunifera (Carnauba) Wax | 8.00 |
| | PERMULGIN 3283 | Ozokerite | 6.60 |
| | ARLACEL™ 582 | Mixture of Sorbitan Isostearate and PEG-2 Hydrogenated Castor Oil and Ozokerite and Hydrogenated Castor Oil | 3.00 |
| D | SOFTISAN® 649 | Bis-diglyceryl Polyacyladipate-2 | 6.50 |
| E | VITAMIN E ACETATE | Tocopheryl Acetate | 1.00 |
| F | TOCOTRIENOL 70% | Tocotrienols and Tocopherol and Oryza sativa (Rice) Bran Oil | 0.02 |
| G | RONAFLAIR® BORONEIGE® SF15 | Boron nitride | 3.00 |

Dispersions of the pigments (UNIPURE YELLOW, UNIPURE RED and A 402 TUDOR ROSEWOOD) were prepared by grinding the solid pigments.

Phase B was prepared as follows: the ingredients were weighed and then melted at about 90-95°C with stirring until the phase was homogeneous.

Phase C was prepared as follows: the ingredients were weighed and then melted at about 80°C.

The composition was prepared as follows: the ingredients of phase A were blended together in a first mixer and stirred until the phase was homogeneous. Phase A was then heated to about 80-85°C. Phase B was heated as disclosed above and added to phase A while homogenizing. The temperature of phases A+B was kept at about 85°C. Phase C was heated as disclosed above and then added to phase A+B while homogenizing. Then, the ingredients of phases D to G were added. The mixture was kept at 85°C, then poured into a lipstick mould and cooled until complete solidification.

### Preparation of the lipstick formulations 2C, 3C and 4-7

The lipstick formulations 2C, 3C and 4-7 were prepared by mixing 0.1 wt.% or 1 wt.% of each of Fomblin^{®} HC/R PFPE, Fomblin^{®} HC/OH-1000 PFPE or Fomblin^{®} HC/P2-1000 PFPE to Formulation 1C prepared as disclosed above, the amounts were based on the total weight of the Formulation 1C as reported in the following Table 1:

**Table 1**

| **Formulation No.** | **Additive** | **Amount** |
|---|---|---|
| 1C(*) | None | - |
| 2C(*) | Fomblin^{®} HC/R PFPE | 1 % w/w |
| 3C(*) | Fomblin^{®} HC/R PFPE | 0.1 % w/w |
| 4(*) | Fomblin^{®} HC/OH-1000 PFPE | 1 % w/w |
| 5(*) | Fomblin^{®} HC/P2-1000 PFPE | 1 % w/w |
| 6 | Fomblin^{®} HC/OH-1000 PFPE | 0.1 % w/w |
| 7 | Fomblin^{®} HC/P2-1000 PFPE | 0.1 % w/w |

| | | |
|---|---|---|
| (*) comparative | | |

### Example 1

The following properties were evaluated for each of the formulations prepared as disclosed above:
- lipstick dropping point was determined using a dropping point thermometer with Ubbelohde pattern;
- stability tests (ageing test) were carried keeping each sample at three different temperatures (namely 4°C, 23°C and 50°C) for three weeks.

The results are reported in the following Table 2:

**Table 2**

| Formulation No. | Dropping point (°C) | Aspect t = 0 | Aspect (t = 3 weeks) | | |
|---|---|---|---|---|---|
| | | | 4°C | 23°C | 50°C |
| 1C(*) | 65 | 1 | 1 | 1 | 1 |
| 2C(*) | 65 | 1 | 1 | 1 | 1 |
| 3C(*) | 66 | 1 | 1 | 1 | 1 |
| 4(*) | 65 | 1 | 1 | 1 | 1 |
| 5(*) | n/a | 0 | n/p | n/p | n/p |
| 6 | 65 | 1 | 1 | 1 | 1 |
| 7 | 66 | 1 | 1 | 1 | 1 |

| | | | | | |
|---|---|---|---|---|---|
| (*) comparative n/a : not available n/p : not performed | | | | | |

The above results showed that only in Formulation No. 5 phase separation was observed, while all the other formulations showed no sediments and/or phase separation. The stability of Formulations 1C, 2C, 3C, 4, 6 and 7 was confirmed after the ageing test, as not separation was observed in the test conditions.

### Example 2 - Evaluation of flowingness and covering power

The flowingness (ease of application) and the covering power were evaluated by 5 persons who were trained before starting the test.

Each person was asked to apply each of the lipstick formulations on the lips and to express with a score from 1 to 4 (wherein: 1 = poor and 4 = excellent) the ease of application and the covering power.

The results, expressed as average results, are summarized in the following Table 3.

**Table 3**

| Formulation No. | Flowingness | | | | | Average | Covering power | | | | | Average |
|---|---|---|---|---|---|---|---|---|---|---|---|---|
| 1C(*) | 2 | 1 | 2 | 3 | 3 | 2.2 | 3 | 4 | 3 | 3 | 3 | 3.2 |
| 2C(*) | 3 | 3 | 3 | 2 | 4 | 3 | 3 | 4 | 4 | 2 | 2 | 3 |
| 3C(*) | 3 | 2 | 3 | 3 | 4 | 3 | 3 | 2 | 3 | 3 | 4 | 3 |
| 4(*) | 3 | 4 | 2 | 2 | 4 | 3 | 3 | 4 | 3 | 3 | 4 | 3.4 |
| 6 | 3 | 2 | 2 | 2 | 2 | 2.2 | 3 | 4 | 4 | 4 | 4 | 3.8 |
| 7 | 4 | 3 | 3 | 1 | 2 | 2.6 | 4 | 4 | 4 | 4 | 4 | 4.0 |

| | | | | | | | | | | | | |
|---|---|---|---|---|---|---|---|---|---|---|---|---|
| (*) comparative | | | | | | | | | | | | |

The above results showed that the flowingness of the lipstick formulation was not impaired by the addition of the functional PFPE polymers as additive.

The above results also showed that the covering power was improved for the formulations comprising the functional PFPE polymers as additive.

### Example 3 - Evaluation of durability

The durability (or long-lasting effect) was evaluated by applying each formulation on the lips of three or seven voluntaries and evaluating the amount and the appearance of the lips after 4 and 8 hours, respectively.

The results are summarized in the following Table 4, wherein:
3 indicates that the colour (and hence the lipstick formulation) is still present and homogeneously distributed on the lips;
2 indicates that the colour is present but mainly in the wrinkles of the lips (i.e. the lipstick formulation caused dehydration of the lips);
1 indicates that the colour is present only in the wrinkles or in the contour of the lips; and
0 indicates no colour on the lips.

**Table 4**

| **No.** | **Evaluation after 4 hours** | | | | | | | **Av.** | **Evaluation after 8 hours** | | | | | | | **Av.** |
|---|---|---|---|---|---|---|---|---|---|---|---|---|---|---|---|---|
| 1C(*) | 2 | 3 | 1 | 2 | 1 | 1 | 1 | 1.57 | 1 | 2 | 0 | 1 | 0 | 0 | 0 | 0.57 |
| 2C(*) | 2 | 2 | 2 | - | - | - | - | 2.0 | 1 | 1 | 1 | - | - | - | - | 1.0 |
| 3C(*) | 2 | 1 | 2 | - | - | - | - | 1.66 | 0 | 1 | 1 | - | - | - | - | 0.66 |
| 4(*) | 1 | 2 | 2 | - | - | - | - | 1.66 | 0 | 1 | 1 | - | - | - | - | 0.66 |
| 6 | 3 | 3 | 2 | 1 | 2 | 2 | 2 | 2.14 | 1 | 1 | 2 | 0 | 1 | 2 | 2 | 1.28 |
| 7 | 2 | 3 | 3 | 1 | 2 | 2 | 2 | 2.14 | 1 | 1 | 2 | 0 | 1 | 1 | 2 | 1.14 |

| | | | | | | | | | | | | | | | | |
|---|---|---|---|---|---|---|---|---|---|---|---|---|---|---|---|---|
| (*) comparative Av. = average value | | | | | | | | | | | | | | | | |

The above results showed that the durability or long-lasting effect of the lipstick formulation was markedly improved by the use of a functional PFPE polymer as additive, especially in an amount of about 0.1 wt.%. An improvement is also obtained when the amount of the functional PFPE is of 1.0 wt.% based on the total weight of the formulation.

### Example 4 - Adhesion test

The adhesion test (no transfer of the colour) was carried out by applying each lipstick formulation twice on the lips of three or seven voluntaries. After 30 minutes from the application, a paper sheet was applied onto the lips of each volunteer for 5 seconds. The stain on the paper sheet was evaluated.

The results are summarized in the following Table 5, wherein:
0 indicates an evident lip mark
1 indicates a partial lip mark
2 indicates a light halo
3 indicates no stain

**Table 5**

| **No.** | **Evaluation** | | | | | | | **Average** |
|---|---|---|---|---|---|---|---|---|
| 1C(*) | 0 | 1 | 1 | 0 | 0 | 0 | 1 | 0.43 |
| 2C(*) | 0 | 0 | 1 | - | - | - | - | 0.33 |
| 3C(*) | 1 | 2 | 0 | - | - | - | - | 1.0 |
| 4(*) | 0 | 1 | 0 | - | - | - | - | 0.33 |
| 6 | 2 | 3 | 2 | 1 | 2 | 2 | 1 | 1.86 |
| 7 | 0 | 2 | 1 | 2 | 1 | 1 | 2 | 1.29 |

| | | | | | | | | |
|---|---|---|---|---|---|---|---|---|
| (*) comparison | | | | | | | | |

The above results showed that the adhesion test was notably improved by the use of a functional PFPE polymer as additive in an amount of about 0.1 wt. %.

## Claims

1. A method for colouring the lips, which comprises applying to the lips a lipstick formulation [formulation F], said formulation F comprising at least one functional (per)fluoropolyether polymer [polymer P] and a pigmented base.

2. The method according to claim 1, wherein polymer P comprises a (per)fluoropolyether chain [chain (R_{pf})] having two chain ends [chains (Rₑ)], wherein at least one chain (Rₑ) bears at least one functional group.

3. The method according to claim 2, wherein chain (Rp_{f}) is a chain of formula:
-O-D-(CFX^{#})_{z1}-O(R_{f})(CFX^{*})_{z2}-D^{*}-O-
wherein
z1 and z2, equal or different from each other, are equal to or higher than 1;
X^{#} and X^{*}, equal or different from each other, are -F or -CF₃, provided that when z1 and/or z2 are higher than 1, X^{#} and X^{*} are -F;
D and D*, equal or different from each other, are an alkylene chain comprising from 1 to 6 and even more preferably from 1 to 3 carbon atoms, said alkyl chain being optionally substituted with at least one perfluoroalkyl group comprising from 1 to 3 carbon atoms;
(R_{f}) comprises, preferably consists of, repeating units R°, said repeating units being independently selected from the group consisting of:
(i) -CFXO-, wherein X is F or CF₃;
(ii) -CFXCFXO-, wherein X, equal or different at each occurrence, is F or CF₃, with the proviso that at least one of X is -F;
(iii) -CF₂CF₂CW₂O- wherein each of W, equal or different from each other, are F, Cl, H;
(iv) -CF₂CF₂CF₂CF₂O-;
(v) -(CF₂)ⱼ-CFZ-O- wherein j is an integer from 0 to 3 and Z is a group of general formula -O-R(_{f-a})-T, wherein R(_{f-a}) is a fluoropolyoxyalkene chain comprising a number of repeating units from 0 to 10, said recurring units being chosen among the following : -CFXO- , -CF₂CFXO-, -CF₂CF₂CF₂O-, -CF₂CF₂ CF₂CF₂O-, with each of X being independently F or CF₃ and T being a C₁-C₃ perfluoroalkyl group.

4. The method according to claim 2 or 3, wherein one chain (Rₑ) bears at least one functional group and the other chain (Rₑ) bears a neutral group selected from -CF₃, -C₂F₅, -C₃F₇, -CF₂Cl, -CF₂CF₂Cl and -C₃F₆Cl.

5. The method according to any one of claims 2 to 4, wherein both chains (Rₑ) bear at least one functional group.

6. The method according to any one of claims 2 to 5, wherein the at least one functional group is selected from the group consisting of hydroxy group and phosphate group.

7. The method according to any one of claims 2 to 6, wherein both chains (Rₑ) bear one hydroxy group or both chains (Rₑ) bear one phosphate group.

8. The method according to any one of the preceding claims, wherein polymer P is in an amount of from 0.001 to less than 1 wt.%, more preferably from 0.05 to 0.5 wt.% based on the total weight of formulation F.

9. The method according to any one of the preceding claims, wherein the pigmented base comprises ingredients selected from the group comprising:
dyes and colorants; moisturizers; UV absorbers; fillers; antioxidants;
conditioning agents; thickeners; flavouring agents; botanical extracts;
anti-microbial agents; anti-irritants agents; chelating agents; preservatives; pH adjusters; humectants; fragrances; essential oils; lubricants; solidifiers (e.g. waxes such as beeswax, carnauba wax or candelilla wax); viscosity modifiers (e.g. polyethylene); and silicone containing compounds.

10. Use of a functional (per)fluoropolyether polymer [polymer P] as additive in a lipstick formulation [formulation F].

11. The use according to claim 9, wherein polymer P is as defined in any one of preceding claims 2 to 7.

12. A lipstick formulation [formulation F] comprising
from 0.001 to less than 1 wt.% based on the total weight of formulation F of at least one functional (per)fluoropolyether polymer [polymer P] and
up to 100 wt.% based on the total weight of formulation F of a pigmented base.

13. The formulation F according to claim 12, wherein polymer P is as defined in any one of the preceding claims 2 to 7.

14. A kit comprising a container having a lid containing a lipstick formulation as defined in claim 12.
